# EUROPEAN PATENT SPECIFICATION

(11) **EP 0 540 887 B1**
(45) Date of publication and mention of the grant of the patent: **09.08.2000**
(21) Application number: 92117089.0
(22) Date of filing: 07.10.1992
(51) Int. Cl.: A61B 17/34

(54) **Safety trocar**
Sicherheitstrokar
Trocart de sécurité

(30) Priority: 04.11.1991 US 787183
(43) Date of publication of application: 12.05.1993
(73) Proprietor: AMERICAN CYANAMID COMPANY, Wayne, NJ 07470-8426 (US)
(72) Inventor: Borgia, Julian F., Danbury, Connecticut 06811 (US); Allen, William J., Stratford, Connecticut 06497 (US); Stein, Jeffrey A., Milford, , Connecticut 06460 (US)
(74) Representative: Wächtershäuser, Günter, Prof. Dr.

(56) References cited:
- EP-A- 0 135 364
- EP-A- 0 479 130
- EP-A- 0 495 633
- EP-A- 0 499 457
- EP-A- 0 511 676

## Description

The present invention relates to a surgical instrument, commonly called a "trocar instrument" or "device," or simply a "trocar," that is used to pierce the wall of an anatomical cavity thereby forming a passageway providing communication with the inside of the cavity. Other medical instruments such as endoscopes, arthoscopes, and operating instruments can thereafter be inserted through the passageway to perform various medical procedures within the anatomical cavity.

EP-A-O 479 130 discloses a safety trocar in which the cutting tip is withdrawn into the cannula in response to counter force being removed from the cutting tip. The counter force is removed from the cuffing tip when the tip enters a body cavity, and is sensed by springs. Accordingly, the sensing is remote from the distal end of the cannula and the tip and thus more susceptible to error through distance. It is desired to sense the counter force closer to the distal end of the cannula and lessen the chance for error.

EP-A-O 499 457 discloses a safety trocar. After the trocar is advanced through a wall of a body cavity and reduces drag on the sleeve, the sleeve is automatically advanced distally. When the sleeve advances distally, the obturator retracts, thereby withdrawing the cutting tip into the cannula.

EP-A-O 135 364 discloses a safety trocar in which a protective shield is biased after a piercing tip has passed through the wall of a body cavity. The protective shield is biased to cover blades and protect against any further puncturing.

EP-A-O 511 676 discloses a safety trocar in which a piercing tip pierces a body wall and a distal end of a cannula is brought into contact with a body wall. The contact forces the cannula proximally until the cannula advances into the body cabity. Then, the absence of a counter force against the distal end of the cannula triggers a mechansim which automatically withdraws the tip into the cannula.

Accordingly, none of the prior art cited shows a counter force sensing portion located between an extreme end of a trocar point and the distal end of a cannula which is desirable for the reasons above.

Surgical techniques using trocar devices to pierce anatomical cavity walls have recently gained great favor in the expanding field known as "least invasive surgery." Such techniques have been widely employed, for example, in gall bladder surgery and their use for other types of operations is actively being explored and implemented. These methods are desirable because the passageway formed by the trocar is small and neat. Therefore, the major trauma associated with large surgical incisions, used to perform certain operations in the past, can be avoided.

The present invention provides an improved safety trocar instrument that is well suited to least invasive surgical techniques. By its design the safety trocar instrument of the present invention not only avoids the trauma that results when large incisions are made in an anatomical cavity wall, but also reduces the chance that unintended and unwanted trauma will result particularly after the instrument penetrates the wall.

In its elemental form, a trocar is a device comprising an elongated shaft of, for example, surgical steel having a sharpened blade or point. Typically, least invasive surgery using such a device is performed first by inserting a fine surgical or "Veress" needle through the cavity wall and thereafter injecting a fluid into the cavity to insufflate it and separate the cavity wall, including muscle and the peritoneum in the case of the abdomen, from other internal organs like the heart, stomach, and major blood vessels. The sharpened point of the trocar is then placed against the cavity wall and urged to pierce it by manually applying pressure to the proximal end of the shaft. An outer sleeve or "cannula" may be slid over the shaft through the wound created by the sharp point. The sleeve permits the shaft to be withdrawn from the cavity wall and maintains the passageway into the cavity. Observation and surgical instruments can then be introduced into the cavity through the sleeve.

Safety trocars described in the prior art have certain inherent drawbacks. First, because the piercing tip of the trocar blade is generally shielded when the instrument is placed against the anatomical cavity wall, it is necessarily shielded from the surgeon's view. Therefore, the surgeon cannot be certain that the tip will puncture the wall at the precise location desired. Moreover, after the piercing tip has penetrated the cavity wall, it must protrude a further substantial distance into the anatomical cavity before the inner sleeve or shield is released again to cover the tip. Thus, a substantial period remains during which the tip is exposed and may injure internal organ structures. Since the inner sleeve or shield and outer sleeve may remain in the cavity after the trocar is removed, they often project a substantial distance into the cavity. Thus the available space in the cavity within which the surgeon can work is reduced.

Therefore, still additional improvement to safety trocar instrument design would be greatly beneficial to the surgical community.

It is a principal object of the present invention to provide an improved safety trocar instrument that mitigates the problems associated with prior art devices and has a safety shield that projects forwardly to surround a sharp trocar point after the point and distal end of the shield penetrate an anatomical cavity wall.

It is an additional principal object of the present invention to provide a self-contained, mechanically actuated safety trocar instrument in which a sharp trocar point is retracted into a surrounding sleeve when the sharp point has penetrated an anatomical cavity wall. The invention thereby results in a substantial improvement over known prior art devices.

These and other objects are achieved by the present invention, which includes a main body that supports an outer sleeve or cannula.

In the first alternative embodiment, the main body is configured to mate with a trocar subassembly that includes a plunger head and a main housing having a trocar. The trocar has a sharp point.

The safety trocar instrument in accordance with the first alternative this preferred embodiment is assembled by mating the main body-cannula subassembly with the trocar subassembly such that the trocar is received coaxially within the cannula. The trocar is urged by a retraction spring to a withdrawn rest position with the point surrounded by the distal end of the cannula.

In the first alternative embodiment, the instrument is armed to pierce an anatomical cavity wall by manually pressing the plunger head of the trocar subassembly into the main body until the sharp trocar point projects beyond the distal end of the cannula with the trocar in a fully extended position. The trocar is initially held in such attitude by an internal latching mechanism residing in the plunger head and main housing of the trocar subassembly. However, the trocar is linked to the latching mechanism through a lost-motion coupling that permits it to be urged backwardly into the housing and plunger head, to an intermediate retracted position, against the force of a pressure spring. The point is nevertheless exposed when the trocar is in the intermediate position.

Once armed, the trocar of the first alternative embodiment is used to pierce an anatomical cavity wall by pressing the exposed point of the trocar against the wall at precisely the desired location. During this operation, pressure against the trocar point urges the trocar backwardly through the lost-motion coupling to the intermediate position in a first stage to prepare a trip mechanism for disarming the latching mechanism. When the cavity wall has been completely penetrated, pressure on the trocar point is relieved permitting it again to be returned through the lost-motion coupling by the pressure spring to its fully extended position. The return of the trocar point causes the trip mechanism to fully disarm the latch mechanism thereby releasing the trocar and permitting it to be retracted to its rest position by the retractor spring with its sharp point surrounded by the distal end of the cannula. The trocar subassembly can then be removed leaving the cannula in the anatomical cavity wall to provide communication with the inside of the cavity.

In the second alternative embodiment, the main body is configured to mate with a trigger sleeve and trocar subassembly that includes a main housing and a plunger head in which a trigger sleeve is axially reciprocally mounted. The trigger sleeve is urged to a rest position projecting outwardly from the main housing. A trocar having a sharp point is coaxially received within the trigger sleeve for reciprocal movement and is urged to a retracted rest position with the sharp point surrounded by the distal end of the sleeve.

The safety trocar instrument in accordance with the second alternative embodiment is assembled by mating the main body-cannula subassembly with the trigger sleeve-trocar subassembly with the inner sleeve and trocar received coaxially within the cannula.

The second alternative embodiment, the instrument is armed to pierce an anatomical cavity wall by manually pressing the plunger head of the trigger sleeve-trocar subassembly into the main body until the distal end of the trigger sleeve projects slightly beyond the distal end of the cannula and thereafter the sharp trocar point projects beyond the distal end of the trigger sleeve. The trocar is latched in such attitude by an internal latching mechanism residing in the plunger head and main housing of the trigger sleeve-trocar subassembly, but the trigger sleeve remains free to reciprocate by being urged back into the cannula. The trocar instrument is then used to pierce an anatomical cavity wall by pressing the exposed point of the trocar against the wall at precisely the desired location. After the point begins to penetrate the wall, the distal end of the trigger sleeve encounters the outer surface of the wall. The resistance of the wall thereafter causes the trigger sleeve to be urged inwardly into the cannula to a retracted position in a first stage preparatory to disarming the latching mechanism. When the cavity wall has been completely penetrated pressure on the trigger sleeve is relieved permitting it again to be urged back to its rest position. The return of the trigger sleeve then fully disarms the latch mechanism thereby releasing the trocar shaft and permitting it to be retracted to its rest position with its sharp point surrounded by the trigger sleeve and cannula. The trocar shaft and trigger subassembly can then be removed leaving the cannula in the anatomical cavity wall to provide communication with the inside of the cavity.

Thus the present invention provides a safety trocar instrument in which the sharp point of the trocar is retracted into a surrounding shield structure. Since retraction occurs immediately upon entry of the trocar point into the anatomical cavity, there is a reduced likelihood of injury to internal organs. Moreover, since retraction occurs promptly at that time, little of the device remains in the cavity after penetration to infringe upon the surgeon's work area. Still further, the trocar point is exposed to the surgeon's view at the start of penetration so that the surgeon can precisely position it at the desired cavity wall location. Thus the safety trocar instrument of the present invention is a substantial improvement over designs of the type disclosed in the prior art.

The present invention is also entirely self-contained and mechanically actuated. Therefore, it is not affected by electrical power failures or interruptions nor does it depend on cumbersome ancillary electrical equipment. And since no wire connections to such ancillary equipment are required, they are not present to interfere with the surgeon's work.

These and other objects, aspects, features, and advantages of the present invention will become apparent from the following detailed description taken in conjunction with the accompanying drawings.

### FIRST EMBODIMENT DRAWINGS

Figures 1A to 1F are a sequence of perspective views of the preferred embodiment of the safety trocar instrument of the present invention showing it at various stages during use;
Figure 2 is a vertical cross-sectional view of the safety trocar instrument of the present invention shown in its assembled rest condition;
Figures 3A to 3D are a sequence of vertical cross-sectional views of the safety trocar instrument of the present invention showing it being armed and operated through initial piercing of an anatomical cavity wall; and
Figures 4A to 4D are a sequence of vertical cross-sectional views of the safety trocar instrument of the present invention showing the trocar retraction operation and the trocar subassembly being removed from the main body-cannula assembly.

### DESCRIPTION OF THE FIRST EMBODIMENT

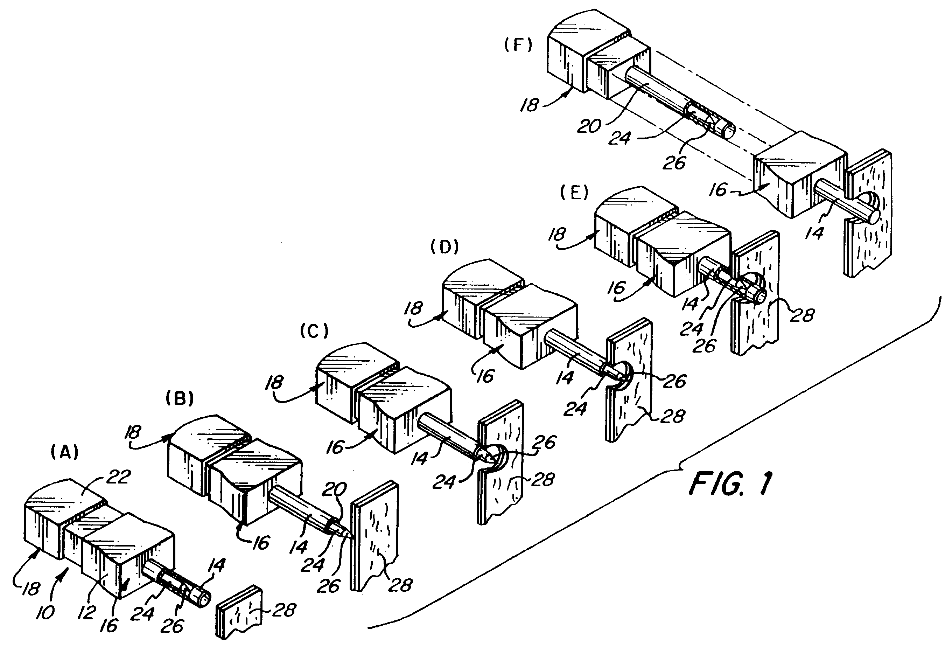

Figures 1A though 1F diagrammatically show the safety trocar instrument in accordance with first alternative embodiment of the present invention as it appears in various stages of use. More particularly, this safety trocar instrument, generally indicated at 10, includes a main body 12 having an outer tubular sleeve or cannula 14 projecting from it. The main body-cannula subassembly, generally indicated at 16, is configured to mate with a trocar subassembly generally indicated at 18, that includes a sleeve 20, mounted in a housing 22, and an elongate trocar 24, having a sharp point 26 and being mounted for axial reciprocal movement in the sleeve 20 but being urged to a retracted position therein.

In the assembled rest position of the instrument 10 shown in Figure 1A, in which the sleeve 20 and cannula 14 are partly broken away to show the location of the trocar point 26, the trocar subassembly 18 is mated with the main body-cannula subassembly 16 such that the coaxially arranged trocar 24 and sleeve 20 are in turn received coaxially within the cannula 14. As can be seen, in this rest position the distal end of the sleeve resides entirely within the distal end of the cannula and the sharp point 26 of the trocar resides within the distal end of the sleeve 20. Thus, in the rest position the sleeve and cannula 14 shield the trocar point 26.

The assembled instrument is armed, as shown in Figure 1B, to pierce an anatomical wall 28 by manually squeezing the trocar subassembly 18 into the main body-cannula subassembly 16. This operation causes the sharp trocar point 26 to project beyond the distal ends of both the sleeve 20 and cannula 14. The trocar is latched in this fully extended position by an internal latching mechanism but is permitted partially to retract from the fully extended position by a lost-motion coupling. Both the latching mechanism and lost-motion coupling will be described in detail below. Thus the trocar point is exposed in preparation for puncturing the cavity wall 28.

As shown in Figures 1B and 1C, the trocar point 26 is visible to a surgeon so that it can be precisely positioned at the desired location on an anatomical cavity wall 28 for the intended puncture wound. As the point begins penetration, counterforce exerted by the wall 28 urges the trocar 24 back toward, but not withdrawn into, the distal end of the cannula to an intermediate retracted position as permitted by the lost-motion coupling. In this intermediate position, a trip mechanism is prepared to disarm the internal latching mechanism.

Figure 1D shows the state of the trocar instrument 10 in which both the trocar point 26 and the distal ends of the sleeve 20 and cannula 14 have cleared the inside surface of the wall 28. Accordingly, the counterforce exerted by the wall on the trocar is relieved permitting it again to be projected to its fully extending position. This action causes the trip mechanism to disarm the internal latch mechanism permitting the trocar to be retracted to its rest position with the trocar point 26 shielded within the sleeve 20 and cannula 14 distal ends, as shown in Figure 1E. In Figure 1E the sleeve 20 and cannula 14 are shown partly broken away for clarity as in Figure 1A.

Finally, as shown in Figure 1F, the trocar subassembly 18 can be withdrawn from the main body-cannula subassembly 16 with the cannula 14 remaining in the puncture wound in the wall 28. The cannula thus provides a passage through the cavity wall into the cavity interior.

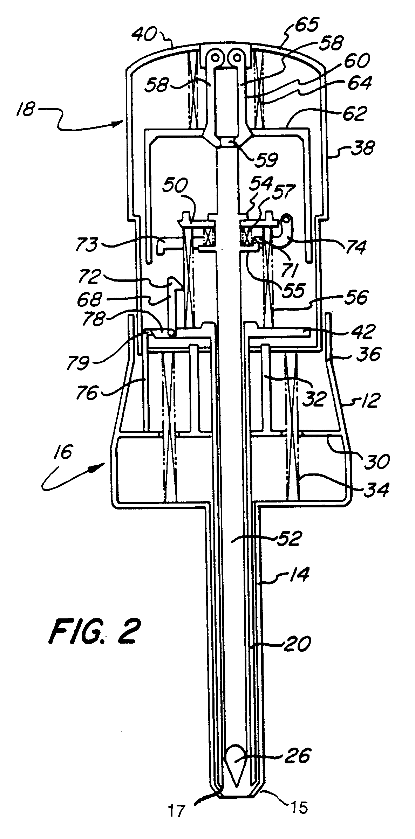

The specific structure of the safety trocar instrument 10 in accordance with a preferred embodiment of the present invention will now be described with reference to Figure 2, which is a vertical cross-sectional view thereof. The trocar device includes the main body 12 having the cannula 14 extending therefrom. The main body 12 is formed with an intermediate partition 30, an upwardly projecting stop 32 on the partition 30, and a captured compression spring 34. The main body 12 is also formed with a generally rectangularly shaped socket 36 projecting upwardly from the partition 30.

The trocar subassembly 18 is configured to mate with main body-cannula subassembly 16 and includes a main housing 38 having an integrally formed plunger head 40, and the sleeve 20, which has a radial flange 42 at its proximal end and is mounted for reciprocal movement within the housing 38. The bottom of the main housing 38 is rectangularly shaped to be telescopically received in the socket 36.

The trocar 24 is mounted for axial reciprocal movement within the trocar subassembly and includes a shaft 52 having radially projecting upper and lower flanges 54 and 55 near its proximal end and the sharp point 26 at its distal end. The trocar shaft 52 is coaxially received within the sleeve 20. A stop plate 50 is received about the trocar shaft 52 between the upper and lower flanges 54 and 55 and is urged upwardly into contact with the upper radial flange 54 by a pressure spring 57. The stop plate 50, pressure spring 57, and flanges 54 and 55 constitute a lost-motion coupling, the function of which will be described in further detail below.

The trocar 24 is urged to a retracted rest position by a retractor spring 56 compressed between the lower side of the stop plate 50, which engages the upper flange 54, and the sleeve flange 42. The trocar 24 is also stopped in this retracted rest position by a pair of pivotable pushers 58 mounted in the plunger head 40 that, when closed in the radial direction, engage the proximal end 59 of the trocar shaft 52, which has a reduced diameter. The pivotable pushers 58 each have a downwardly, radially outwardly tapered outer cam surface 60 that is engaged by an inner aperture in an embracing ring 62 mounted in the plunger head 40. The ring 62 is urged downwardly by a plunger spring 64 that is compressed between it and the inner surface of the top 65 of the plunger head 40. The embracing ring 62, which thus constitutes a cam driver, urges the pivotable pushers 58 radially together by engaging the tapered outer pusher surfaces 60.

In addition, the trocar subassembly incorporates a latch mechanism the function of which was generally described above. Now, in detail, this latch mechanism includes latch means 66 in the form of a pawl 68 mounted for pivoted movement on the sleeve flange 42, and an upstanding tab 76 projecting upwardly from the intermediate partition 30 in the main body through a hole in the bottom of the main housing. The pawl 68 is formed with a hook 72 at its upper end, which can override and engage an upper edge of the stop plate 50, and a foot 78 at its lower end that can engage a catch 79 at the upper end of the tab 76.

A trip mechanism 75 for the latch mechanism, comprising the tab 76 and pawl 68, includes an arm 74 pivotably mounted in the plunger head. The arm 74 has a tab 71, which can be engaged by the lower edge of the lower flange 55 to cause the arm 74 to pivot in the counterclockwise direction, and a finger 73, which can engage the foot 78 of pawl 68. It is these latch and trip mechanisms, in cooperation with the trocar flanges 54 and 55 and the stop plate 50, that determine the sequence of operations of the trocar instrument described above with reference to Figures 1A to 1F.

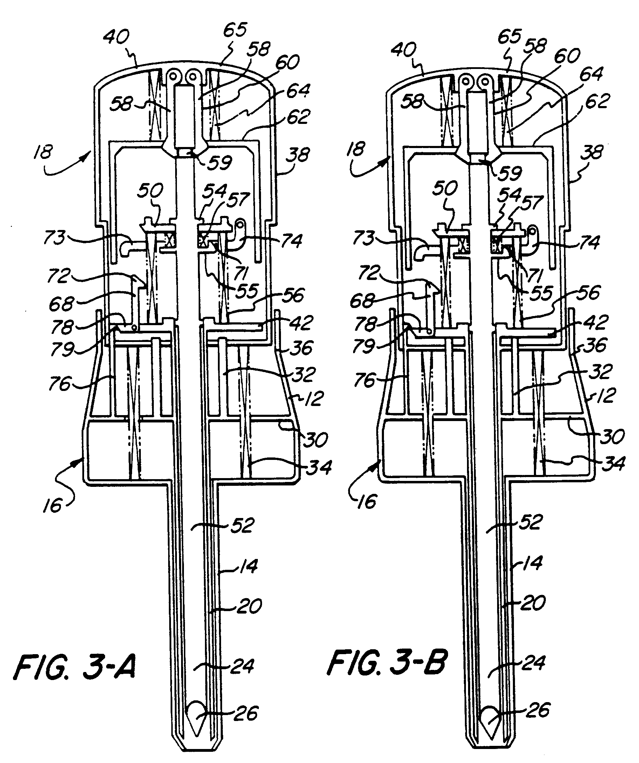

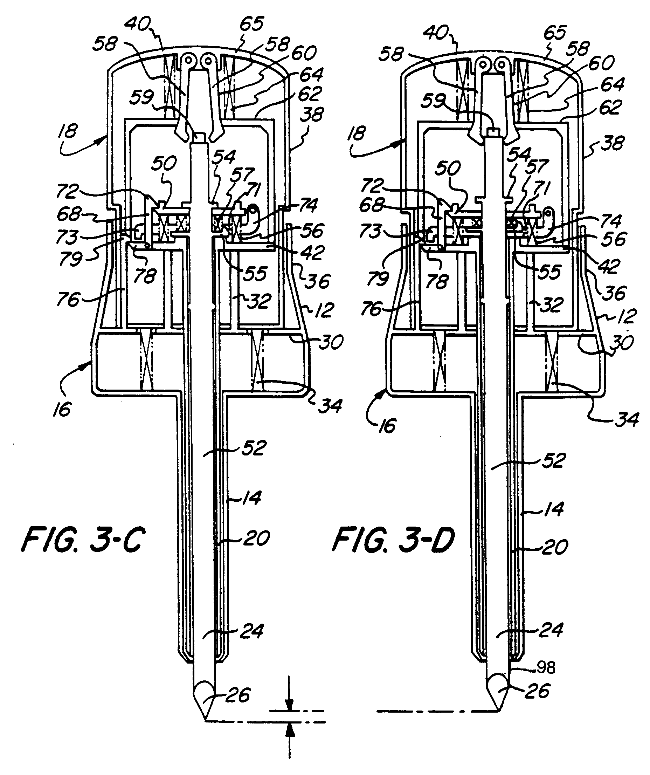

More particularly, Figures 3A through 3D show the sequence of movements of the various elements of the safety trocar instrument described above from the rest position to arming of the instrument, and, in turn, to initial penetration of the anatomical cavity wall.

In the initial rest position shown in Figure 3A, which is substantially the same as Figure 2, the trocar subassembly 18 is inserted into the main body-cannula subassembly 16 with the sleeve 20 and trocar shaft 52 coaxially received within the cannula 14. The distal ends of the sleeve 20 and cannula 14 terminate at nearly the same axial location, with the main housing 38 being urged outwardly from the main body 12 to its rest position by the relaxed compression spring 34.

When the device is to be used, the trocar subassembly 18 is manually squeezed into main body-cannula subassembly 16 thereby compressing the captured spring 34, until the catch 79 of the tab 76 catches the foot 78 of the pawl 68 with the sleeve flange 42 abutting the stop 32, which projects through holes in the bottom of the main housing 38. At this stage, the trocar shaft is moved downwardly a small distance by the pushers 58, but the trocar point 26 remains within both the sleeve 20 and cannula 14, as shown in Figure 3B.

Further depression of plunger head 40 relative to the main housing 38 causes the pivotable pushers 58 to push the trocar shaft 54 axially downwardly further until the hook 72 of the pawl 68 overrides and catches the edge of the stop plate 50 thereby compressing the retractor spring 56. At this stage the trocar point 26 projects to a fully extended position beyond the distal ends of both the sleeve 20 and cannula 14, as shown in Figure 3C. Those distal ends also become substantially coextensive.

Ultimately, complete depression of plunger head 40 causes the sidewall of the embracing ring 62 to engage the periphery of the sleeve flange 42, which as noted has come to rest on the top of a stop 32 projecting upwardly from the intermediate partition 30. Therefore, further downward movement of the plunger head 40 causes the embracing ring 62 to move upwardly relative to the pushers 58, which are then permitted to spread radially outwardly and release the proximal end 59 of the trocar shaft 52. In this configuration, shown in Figure 3C, the trocar instrument is armed and ready to pierce an anatomical cavity wall.

The instrument can then create a puncture wound by pressing the point 26 of the trocar 24 against the cavity wall. As the point begins its entry, resistance or counterforce exerted by the wall causes the lower portion 98 of trocar shaft 52 to be urged inwardly into cannula 14 and sleeve 20, against the force of the pressure spring 57, to an intermediate retracted position until the edge of the lower flange 55 overrides the tab 71 of arm 74, as shown in Figure 3D. The upper and lower flanges 54 and 55 in cooperation with the pressure spring 57 permit this lost trocar motion 96 relative to the stop plate 50.

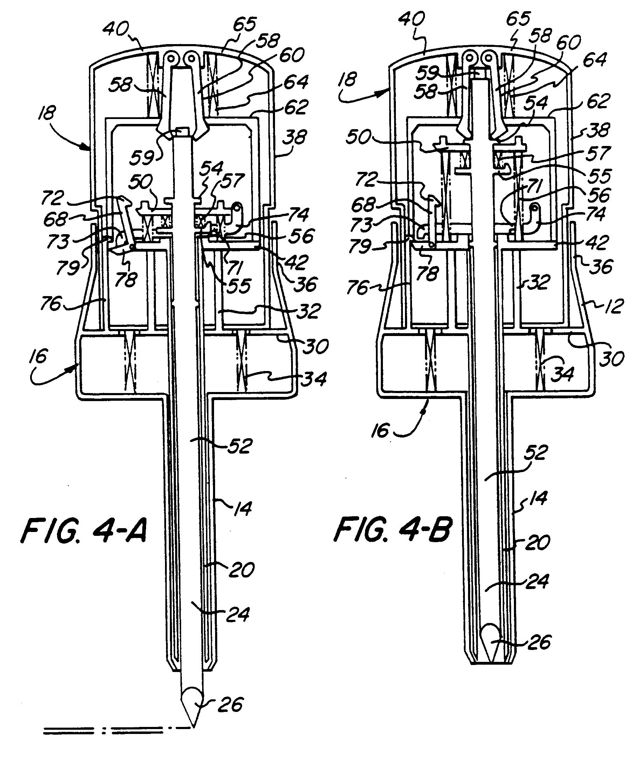

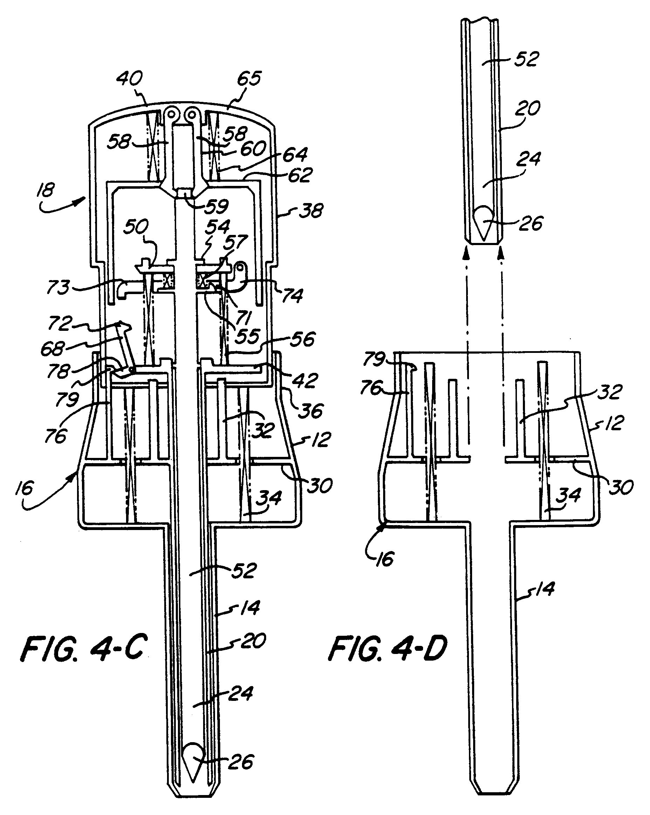

Figures 4A to 4D show the sequence of movement of the various elements of the safety trocar instrument as the cavity wall is penetrated and retraction of the trocar point 26 is subsequently triggered. As depicted in Figure 4A, counterforce against the point 26 of the trocar shaft 52 is relieved when the trocar and cannula 14 clear the inner surface of the cavity wall. Therefore, the pressure spring 57 may urge the trocar shaft 52 downwardly again from the intermediate position toward the fully extended position. This motion of the trocar causes the arm 74 to be pivoted in the counterclockwise direction by engagement of the edge of the lower flange 55 with the tab 71. The extreme end of the arm 74 then depresses the foot 78 of pawl 68 causing it also to pivot in the counterclockwise direction to disengage its hook 72 from the edge of the stop plate 50, thereby releasing the stop plate 50. The retractor spring 56 compressed between the flange 42 and the stop plate 50 then urges the trocar shaft 52 upwardly into the sleeve 20 and cannula 14 to its withdrawn rest position with the point shielded within the distal ends of both, as depicted in Figure 4B.

When the plunger head 40 is thereafter manually released, it may move upwardly within the main housing 38 under the influence of compressed spring 34, disengaging the sidewall of the embracing ring 62 from the periphery of the sleeve flange 42. The ring 62 may then be urged downwardly to embrace the pushers 58 and urge them together. The pushers may then again grip the proximal end 59 of the trocar shaft 52, as shown in Figure 4C. As can also be seen there, the pawl 68 can swing about its pivot so that the foot 78 can be disengaged from the catch 79 of the leg 76.

Thereafter the trocar subassembly may be removed from the main body-cannula subassembly such that the cannula remains in the anatomical cavity wall to provide communication with the cavity interior.

It should be noted that the sleeve 20 in the trocar subassembly is long enough in the preferred embodiment to shield the trocar point, particularly when the trocar subassembly is in its rest condition and removed from the main body-cannula subassembly. However, the sleeve 20 may be eliminated if desired since it, by itself, is not necessary to retraction of the trocar point into the distal end of the cannula. If the sleeve is eliminated, the cannula will shield the trocar point after it is retracted following cavity penetration.

### SECOND EMBODIMENT DRAWINGS

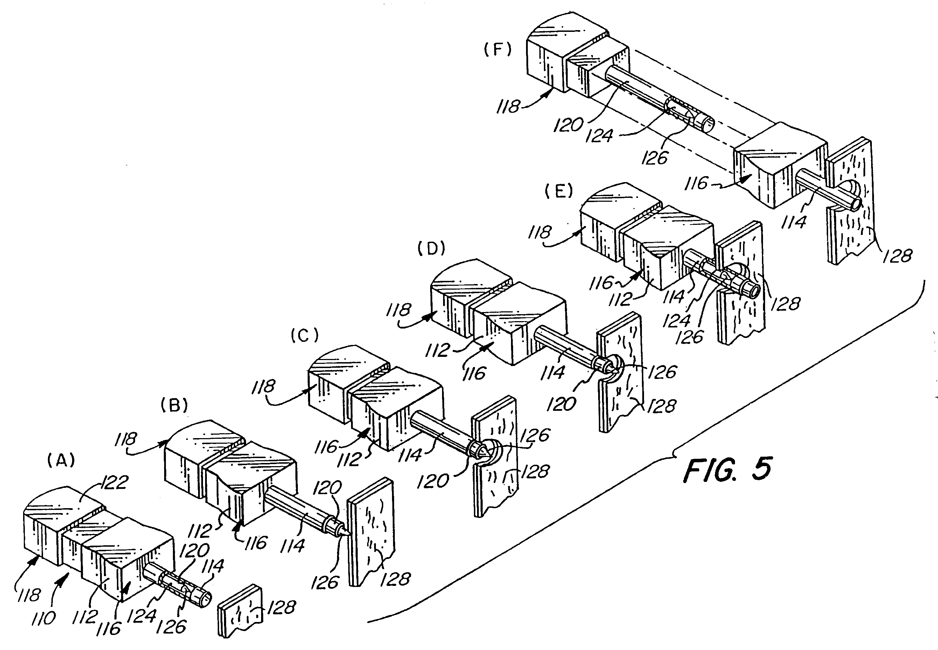

Figures 5A to 5F are a sequence of perspective views of the preferred embodiment of the safety trocar instrument of the present invention showing it at various stages during use;

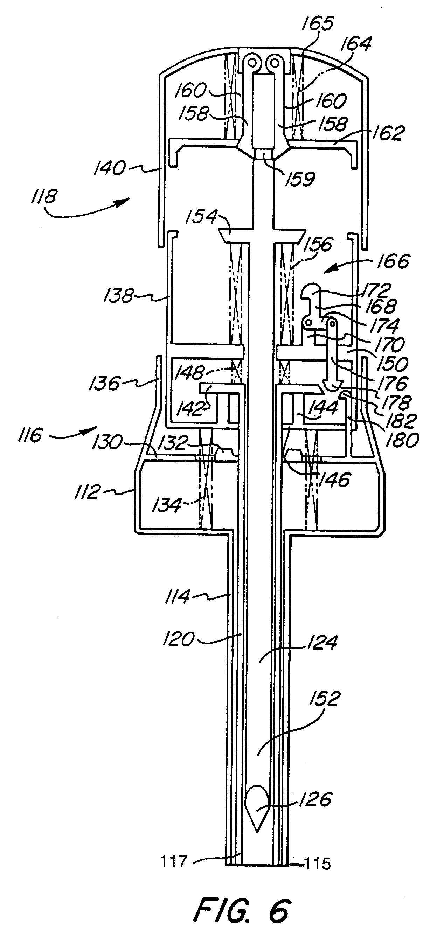

Figure 6 is a vertical cross-sectional view of the safety trocar instrument of the present invention shown in its assembled rest condition;

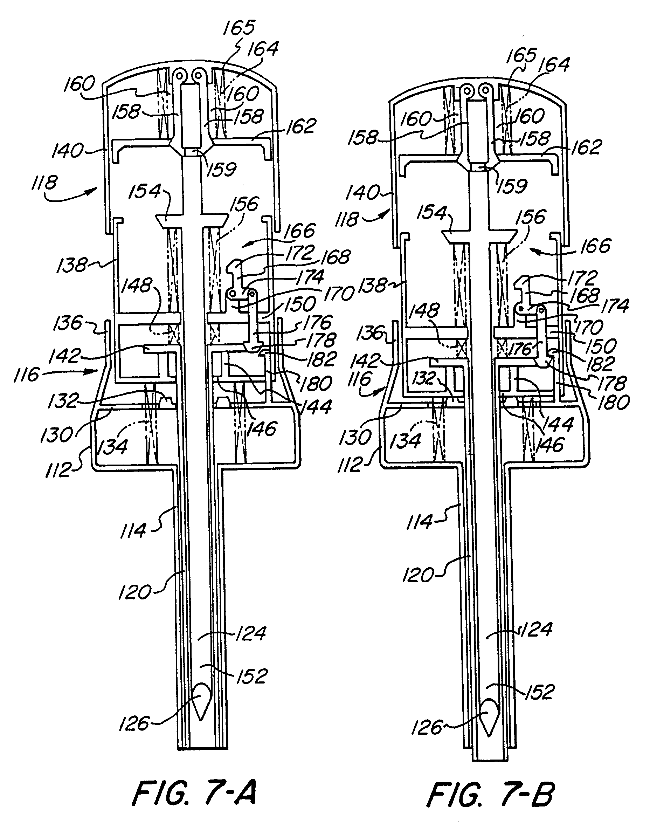

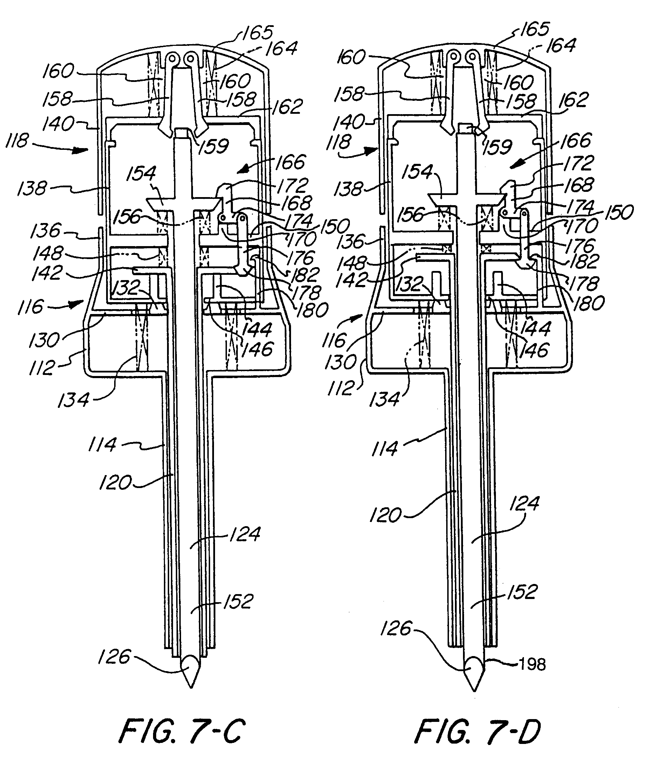

Figures 7A to 7D are a sequence of vertical cross-sectional views of the safety trocar instrument of the present invention showing it being armed and operated through initial piercing of an anatomical cavity wall; and

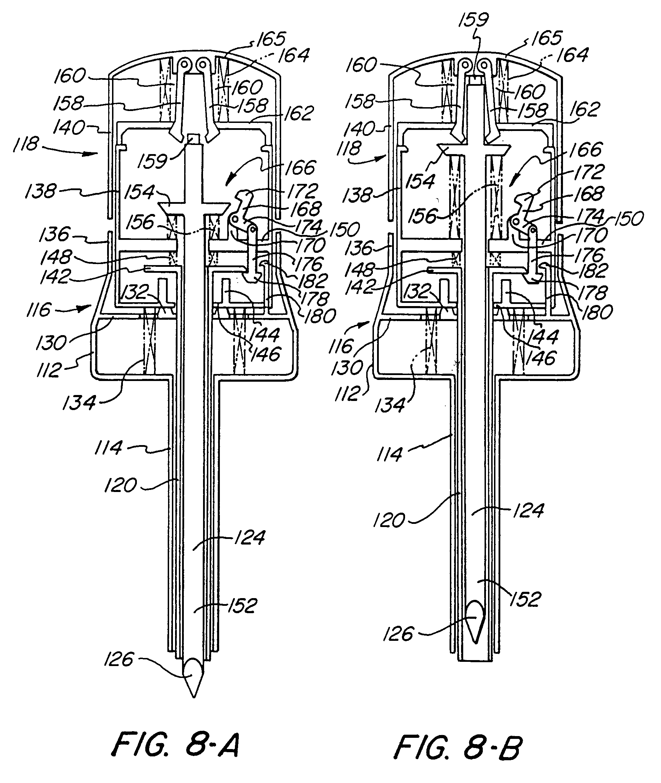

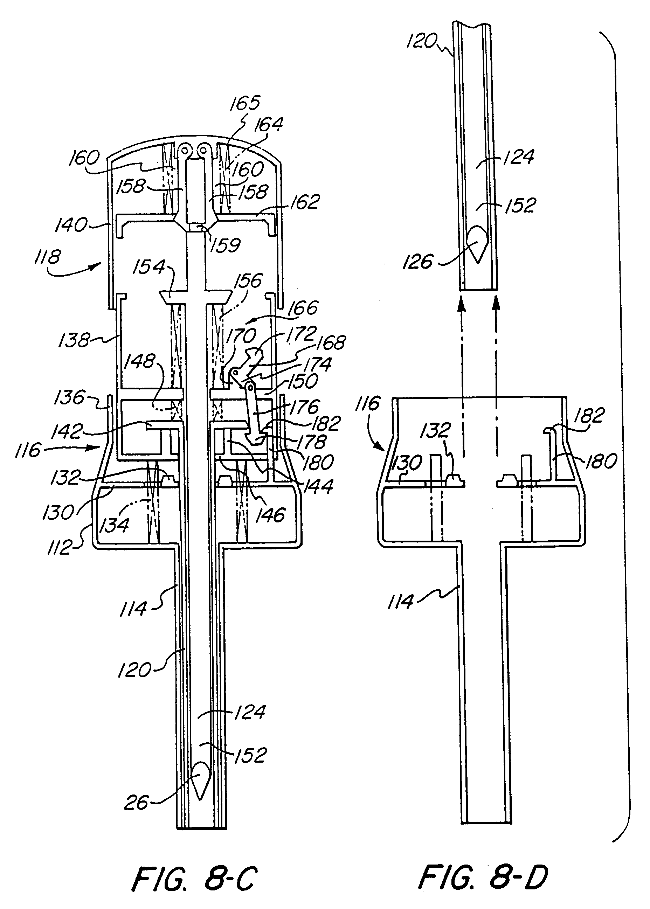

Figures 8A to 8D are a sequence of vertical cross-sectional views of the safety trocar instrument of the present invention showing the trocar retraction operation and the trigger sleeve-trocar subassembly being removed from the main body-cannula assembly.

### DESCRIPTION OF THE SECOND EMBODIMENT

Figures 5A though 5F diagrammatically show the safety trocar instrument in accordance with the second alternative embodiment of the present invention as it appears in various stages of use. More particularly, this safety trocar instrument, generally indicated at 110, includes a main body 112 having an outer tubular sleeve or cannula 114 projecting from it. The main body-cannula subassembly, generally indicated at 116, is configured to mate with a trigger sleeve-trocar subassembly generally indicated at 118, that includes a tubular trigger sleeve 120, mounted for reciprocal movement in a housing 122 and urged to an outwardly extending position therefrom, and an elongate trocar 124, having a sharp point 126 and being mounted for axial reciprocal movement in the trigger sleeve 120 but being urged to a retracted position therein.

In the assembled rest position of the instrument 110 shown in Figure 5A, in which the trigger sleeve 120 and cannula 114 are partly broken away to show the location of the trocar point 126, the trigger sleeve-trocar subassembly 118 is mated with the main body-cannula subassembly 116 such that the coaxially arranged trocar 122 and trigger sleeve 120 are in turn received coaxially within the cannula 114. As can be seen, in this rest position the distal end of the trigger sleeve resides substantially entirely within the distal end of the cannula and the sharp point 126 of the trocar resides within the distal end of the trigger sleeve 120. Thus, in the rest position the trigger sleeve shields the trocar point 126.

The assembled instrument is armed, as shown in Figure 5B, to pierce an anatomical wall 128 by manually squeezing the trigger sleeve-trocar subassembly 118 into the main body-cannula subassembly 116. This operation causes the distal end of the trigger sleeve 120 to project beyond the distal end of the cannula 114 and causes the sharp trocar point 126 to project beyond the distal end of the trigger sleeve 120. The trocar is latched in this projecting position by an internal latching mechanism to be described in detail below. Thus the trocar point is exposed in preparation for puncturing the cavity wall 128.

As shown in Figures 5B and 5C, the trocar point 126 is visible to a surgeon so that it can be precisely positioned at the desired location on an anatomical cavity wall 128 for the intended puncture wound. As the point begins penetration, counterforce exerted by the wall 128 urges the distal end of the trigger sleeve 120 back into the distal end of the cannula to a first position. In this first retracted position, the trigger sleeve is prepared to disarm the internal latch mechanism holding the trocar in its extended position.

Figure 5D shows the state of the trocar instrument 110 in which both the trocar point and the distal ends of the trigger sleeve and cannula have cleared the inside surface of the wall 128. Accordingly, the counterforce exerted by the wall on the trigger sleeve is relieved permitting it again to be projected to its extended position with its distal end protruding beyond the distal end of the cannula. This action of the trigger sleeve disarms the internal latch mechanism permitting the trocar to be retracted to its rest position with the trocar point within the trigger sleeve distal end, as shown in Figure 5E. In Figure 5E the trigger sleeve and cannula are shown partly broken away for clarity as in Figure 5A.

Finally, as shown in Figure 5F, the trigger sleeve-trocar subassembly 118 can be withdrawn from the main body-cannula subassembly 116 with the cannula 114 remaining in the puncture wound in the wall 128. The cannula thus provides a passage through the cavity wall into the cavity interior.

The specific structure of the safety trocar instrument 110 in accordance with a preferred embodiment of the present invention will now be described with reference to Figure 6, which is a vertical cross-sectional view thereof. The trocar device includes the main body 112 having the cannula 114 extending therefrom. The main body 112 is formed with an intermediate partition 130, an upwardly projecting stop 132 on the partition 130, and a captured compression spring 134. The main body 112 is also formed with a generally rectangularly shaped socket 136 projecting upwardly from the partition 130.

The trigger sleeve-trocar subassembly 118 is configured to mate with main body-cannula subassembly 116 and has a main housing 138, a plunger head 140, and the trigger sleeve 120, which is reciprocally mounted within housing 138. The bottom of the main housing 138 is rectangularly shaped to be telescopically received in the socket 136. The plunger head 140 is mounted for reciprocal telescoping movement relative to the housing 138 and the two elements are prevented from disengaging from one another by suitable means (not shown).

The trigger sleeve 120 has a rest position relative to the main housing 138 in which a radial flange 142 at its proximal end is supported by a retainer 144 on the bottom 146 of the housing 138. The trigger sleeve is urged to this rest position by a motion spring 148 compressed between the radial trigger sleeve flange 142 and the underside of an inner horizontal partition 150 formed within housing 138.

The trocar 124 is also mounted for axial reciprocal movement within the trigger sleeve-trocar subassembly and includes a shaft 152 having catch means in the form of a radial flange 154 near its proximal end and the sharp point 126 at its distal end. The trocar shaft 152 is coaxially received within the trigger sleeve 120. The trocar 124 is urged to a retracted rest position by a retractor spring 156 compressed between the upper side of the horizontal partition 150 and the radial flange 154. The trocar 124 is also stopped in this retracted rest position by a pair of pivotable pushers 158 mounted in the plunger head 110 that, when closed in the radial direction, engage the proximal end 159 of the trocar shaft 152, which has a reduced diameter. The pivotable pushers 158 each have a downwardly, radially outwardly tapered outer cam surface 160 that is engaged by an inner aperture in an embracing ring 162 mounted in the plunger head 140. The ring 162 is urged downwardly by a plunger spring 164 that is compressed between it and the inner surface of the top 165 of the plunger head 140. The embracing ring 162, which thus constitutes a cam driver, urges the pivotable pushers 158 radially together by engaging the tapered outer pusher surfaces.

In addition, the trigger sleeve-trocar subassembly incorporates a latch mechanism, indicated generally at 166, the function of which was generally described above. Now, in detail, this latch mechanism 166 includes latch means in the form of a pawl 168 mounted for pivoted movement on a stand 170 projecting upwardly from the inner partition 150 within the housing 138. The pawl 168 is formed with a hook 172 that can engage the top of the flange 154 on the trocar shaft 152. A sidewardly projecting arm 174 is formed on the pawl 168 and carries pivotably mounted trip means 175 in the form of a leg 176 that projects downwardly through the partition 150 and has an enlarged foot 178 at its lower free end. This foot is configured to engage the head 182 of an upstanding tab 180 projecting from the partition 130 in the main body through a hole in the bottom 146 of the housing 138. It is this latch mechanism, in cooperation with the trocar flange 154 and the trigger sleeve flange 142, that determines the sequence of operations of the trocar instrument described above with reference to Figures 5A to 5F.

More particularly, Figures 7A through 7D show the sequence of movements of the various elements of the safety trocar instrument described above from the rest position to arming of the instrument, and, in turn, to initial penetration of the anatomical cavity wall.

In the initial rest position shown in Figure 7A, which is substantially the same as Figure 6, the trigger sleeve-trocar assembly 118 is inserted into the main body-cannula subassembly 116 with the trigger sleeve 120 and trocar shaft 52 coaxially received within the cannula 114. The distal ends of trigger sleeve 120 and cannula 114 terminate at about the same axial location, with the main housing 138 being urged outwardly from the main body 112 to its rest position by the relaxed compression spring 134.

When the device is to be used, the trigger sleeve-trocar subassembly 118 is manually squeezed into main body-cannula subassembly 116 thereby compressing the captured spring 134, until the bottom wall 146 of housing 138 abuts the stop 132. In this position, shown in Figure 7B, the foot 178 of the leg 176 is caught under the head 182 of the tab 180. The distal end of the trigger sleeve 120 projects slightly beyond on the distal end of the cannula 114, but the point 126 of the trocar 124 remains within both trigger sleeve 120 and cannula 114.

Further depression of plunger head 140 relative to the main housing 138 causes the pivotable pushers 158 to push the trocar shaft 154 axially downwardly until the hook 172 of the pawl 168 overrides the flange 154 on the shaft 152. The point 126 of the trocar 124 then projects beyond the distal ends of both the trigger sleeve 120 and cannula 114, as shown in Figure 7C.

Ultimately, complete depression of plunger head 140 causes the periphery 183 of the embracing ring 162 to engage the upper extreme 184 of the housing 138 thereby stopping relative embracing ring-main housing movement. Further downward movement of the plunger head 140 causes the embracing ring 162 to move upwardly relative to the pushers 158 which are then permitted to spread radially outwardly and release the proximal end 159 of the trocar shaft 152. In this configuration, shown in Figure 7C, the trocar instrument is armed and ready to pierce an anatomical cavity wall.

The instrument can then create a puncture wound by pressing the point 126 of the trocar 124 against the cavity wall. After the point begins its entry, the distal end of the trigger sleeve encounters the cavity wall. Resistance or counterforce exerted by the wall causes the trigger sleeve 120 to be urged inwardly into cannula 114, against the force of the motion spring 148, until the edge of the radial trigger sleeve flange 142 overrides the foot 178 of the leg 176, as shown in Figure 7D.

Figures 8A to 8D show the sequence of movement of the various elements of the safety trocar instrument as the cavity wall is penetrated and retraction of the trocar point is subsequently triggered. As depicted in Figure 8A, counterforce against the distal end of the trigger sleeve 120 is relieved when the trigger sleeve and cannula 114 clear the inner surface of the cavity wall so that the motion spring 148 may urge the trigger sleeve 120 downwardly again toward its rest position. This motion of the sleeve causes the radial flange 142 to pull the leg 176 downwardly thereby pivoting the pawl 168 so that it hook 172 is disengaged from the radial flange 154 on the trocar shaft.

The trocar shaft 152 is thereby released so that its point 126 is once again retracted into the trigger sleeve 120 and cannula 114 by the force of the retractor spring 156, as depicted in Figure 8B.

When plunger head 140 is thereafter manually released, it may move upwardly within the main housing 138, under the influence of plunger spring 164, permitting the periphery 183 of the embracing ring 162 to clear the upper extreme of the housing 138 and again embrace the outer tapered sides 160 of pivotable pushers 158 to urge the pushers 158 together. The pushers may then again grip the proximal end 159 of the trocar shaft 152, as shown in Figure 8C. As can also be seen there, the leg 176 can swing about its pivot on the arm 174 so that the foot 178 can be disengaged from both the trigger-sleeve flange 142 and the head 182 of the tab 180. Appropriate biasing means for the latch can be provided to ensure disengagement.

Thereafter the trigger sleeve-trocar subassembly may be removed from main body-cannula subassembly such that the cannula remains in the anatomical cavity wall to provide communication with the cavity interior.

Accordingly, it will be appreciated that the present invention provides an improved safety trocar instrument that retracts a sharpened trocar point into a shielding trigger sleeve as soon as the distal end of the sleeve penetrates an anatomical cavity wall. The instrument may be self-contained and is mechanically actuated. Therefore, reliable operation does not depend on external power supplies or electrical triggering mechanisms.

Although specific embodiments of the present invention have been described above in detail, it will be understood that this description is merely for purposes of illustration. Various modifications of and equivalent structures corresponding to the disclosed aspects of the preferred embodiments in addition to those described above may be made by those skilled in the art without departing from the spirit of the present invention which is defined in the following claims, the scope of which is to be accorded the broadest interpretation so as to encompass such modifications and equivalent structures.

## Claims

1. A safety trocar instrument for piercing a wall of an anatomical cavity, having an interior, to provide communication with the interior of the cavity, said trocar instrument comprising:
(a) a tubular cannula (14, 114) having a distal end (15, 115);
(b) an elongate trocar (24, 124), having a sharp piercing point (26, 126) terminating in an extreme end, reversibly shielded by a sleeve (20, 120) having a distal end (17, 117) mountable for axial reciprocal movement within said cannula (14, 114) between a withdrawn rest position in which said point is received within and shielded by the distal end (15, 115) of said cannula (14, 114) and sleeve (20, 120) and a projecting position in which said point (26, 126) is exposed beyond the distal end (15, 115) of said cannula (14, 114) and sleeve (20, 120);
(c) means for biasing (56, 156) said trocar (24, 124) to its withdrawn rest position;
(d) latch means (66, 166) for latching said trocar (24, 124) in the projecting position in opposition to said biasing means (56, 156) to allow clear unobstructed visibility of the wall of the cavity for precision-piercing thereof;
(e) trip means (75, 175) for tripping said latching means (66, 166) to unlatch said trocar (24, 124) and thereby permitting said trocar (24, 124) to be moved to its withdrawn rest position by said biasing means (56, 156);
(f) mechanical trigger means (57, 55 and 71) mounted within said instrument (10, 110), having a counterforce sensing portion (98, 198) locatable between the extreme end of said trocar point (26, 126) and the distal end (15, 115) of said cannula (14, 114), said sensing portion (98, 198) sensing a counterforce exerted by the wall of the cavity after it has begun to be pierced by said trocar point (26, 126) and sensing relief of such counterforce when said sensing portion (98, 198) has pierced the wall of the cavity, said trigger means (96) being mechanically coupleable to said trip means (75, 175) to cause said trip means (75, 175) to trip said latch means (66, 166) in response to sensing relief of such counter force to protect internal organs; and
(g) plunger means for manually moving said trocar (24, 124) from the withdrawn rest position to the projecting position;
wherein said trigger means comprises a tubular trigger sleeve (20, 120) in which said trocar (24, 124) is coaxially received, and said sensing portion comprises the distal end (17, 117) of said trigger sleeve (20, 120) which is locatable between the extreme of said trocar point (26, 126) and the distal end (15, 115) of said cannula (14, 114), said trigger sleeve (20, 120) being mounted coaxially within said cannula (14, 114) for reciprocal movement between an extended rest position with the trigger sleeve (20, 120) distal end (17, 117) located between the extreme of said trocar point (26, 126) and the distal end (15, 115) of said cannula (14, 114) and a retracted position with the trigger sleeve (20, 120) distal end (17, 117) retracted toward the distal end (15, 115) of said cannula (14, 114).

2. The safety trocar instrument (10, 110) according to Claim 1, wherein said trigger sleeve (20, 120) is mounted to be coupled to said trip means (75, 175) when it is moved to the retracted position and thereafter causes said trip means (75, 175) to trip said latch means (66, 166) when it is returned to its extended rest position.

3. The safety trocar instrument (10, 110) according to Claim 2, further comprising means (56, 156) for urging said trigger sleeve (20, 120) to its extended rest position.

4. The safety trocar instrument (10, 110) according to Claim 1, wherein said trigger means (57, 55 and 71) is comprised of said elongate trocar (24, 124), said trocar being mounted for axial reciprocal movement with said cannula (14, 114) between the withdrawn rest position, the fully extended projecting position, and an intermediate position retracted toward the rest position from the fully extended position in which said point (26, 126) is also exposed beyond the distal end (15, 115) of said cannula (14, 114);
and wherein said trip means (75, 175) couples said trocar (24, 124) to said latch means (66, 166) when said trocar (24, 124) moves from the fully extended position to the intermediate position, and trips said latch means (66, 166) when said trocar (24, 124) returns toward said fully extended position to unlatch said trocar (24, 124) and thereby permit said trocar (24, 124) to be moved to the withdrawn rest position by said biasing means (56, 156).

5. The safety trocar instrument (10, 110) according to Claim 4, further comprising means for urging said trocar (24, 124) from the intermediate position toward the fully extended position when said trocar (24, 124) is latched to said latch means (66, 166).

6. The safety trocar instrument (10, 110) according to Claim 4, wherein said trocar (24, 124) further comprises lost-motion coupling means (98, 198) for coupling said trocar (24, 124) to said latch means (66, 166) and including upper and lower flanges mounted in the region of the proximal end of said trocar (24, 124) and a stop plate mounted on said trocar (24, 124) for reciprocal movement between said upper and lower flanges.

7. The safety trocar instrument (10, 110) according to Claim 1, wherein said plunger means comprises means for gripping a portion of said trocar (24, 124) remote from said piercing point (26, 126) when said trocar (24, 124) is in the withdrawn rest position and for releasing said remote portion when said trocar (24, 124) reaches the fully extended position.

8. The safety trocar instrument (10, 110) according to Claim 1, wherein said trocar (24, 124), said biasing means (56, 156), and said trip means (75, 175) constitute a trocar subassembly (18, 118) that is removable as a unit from said cannula (14, 114).

## Patentansprüche

1. Sicherheits-Trokarvorrichtung zum Durchbohren einer Wand eines anatomischen Hohlraums, die einen Innenbereich aufweist, um ein Einwirken auf das Innere des Hohlraums zu gewährleisten, wobei die Trokarvorrichtung umfaßt:
(a) eine röhrenförmige Kanüle (14, 114) mit einem distalen Ende (15, 115);
(b) einen länglichen Trokar (24, 124) mit einem scharfen Bohrpunkt (26, 126), der sich bis zu einem äußeren Ende erstreckt, der reversibel durch ein kurzes Rohr (20, 120) geschützt ist, das ein distales Ende (17, 117) aufweist, das zur axialen wechselseitigen Bewegung innerhalb der Kanüle (14, 114) zwischen der zurückgezogenen Ruhestellung befestigt werden kann, in der der Bohrpunkt im distalen Ende (15, 115) der Kanüle (14, 114) und des kurzen Rohres aufgenommen und geschützt wird, und mit einer Projektionsstellung, in der der Bohrpunkt (26, 126) über das distale Ende (15, 115) der Kanüle (14, 114) und des kurzen Rohres (20, 120) hinaus exponiert wird;
(c) eine Vorrichtung zum Vorspannen (56, 156) der Hohlnadel (24, 124) in ihre zurückgezogene Ruhestellung;
(d) eine Vorrichtung zum Verschließen (66, 166) der Hohlnadel (24, 124) in der Projektionsstellung gegenüber der Vorrichtung zum Vorspannen (56, 156), damit die deutliche und ungehinderte Sichtbarkeit der Hohlraumwand zum Zwecke der präzisen Durchbohrung ermöglicht wird;
(e) eine Vorrichtung zum Schalten (75, 175) der Schließvorrichtung (66, 166) zur Freigabe der Hohlnadel (24, 124), wobei der Trokar (24, 124) durch die Vorspannvorrichtung (56, 156) in ihre zurückgezogene Ruhestellung versetzt werden kann;
(f) eine mechanische Auslösevorrichtung (57, 55, und 71), die in der Vorrichtung (10, 110) befestigt ist und einen Abschnitt zur Wahrnehmung einer Gegenkraft (98, 198) aufweist, die zwischen dem äußersten Ende des Bohrpunkts (26, 126) und dem distalen Ende der Kanüle (14, 114) angeordnet werden kann, wobei der Abschnitt zur Wahrnehmung der Gegenkraft (98, 198) die Gegenkraft wahrnimmt, die von der Hohlraumwand ausgeübt wird, nachdem das Durchbohren mit dem Bohrpunkt (26, 126) eingesetzt hat, wobei das Nachlassen einer solchen Gegenkraft wahrgenommen wird, wenn der Abschnitt zur Wahrnehmung der Gegenkraft (98, 198) die Hohlraumwand durchbohrt hat und wobei die Auslösevorrichtung (96) mechanisch mit der Schaltvorrichtung (75, 175) verbunden werden kann, damit die Schaltvorrichtung (75, 175) die Schließvorrichtung (66, 166) als Reaktion auf das wahrgenommene Nachlassen einer solchen Gegenkraft betätigen kann, damit innere Organe geschützt werden; und
(g) einen Kolben zur manuellen Bewegung der Hohlnadel (24, 124) von der zurückgezogenen Ruhestellung in die Projektionsstellung, wobei die Auslösevorrichtung eine Auslöseröhre (20, 120) aufweist, in der die Hohlnadel (24, 124) coaxial aufgenommen wird und der Abschnitt zur Wahrnehmung mit dem distalen Ende (17, 117) der Auslöseröhre (20, 120) versehen ist, die zwischen dem äußersten Ende des Bohrpunkts (26, 126) und dem distalen Ende (15, 115) der Hohlnadel (14, 114) angeordnet werden kann, wobei die Auslöseröhre (20, 120) coaxial in der Hohlnadel (14, 114) befestigt ist, um eine Umkehrbewegung zwischen einer ausgeweiteten Ruhestellung mit dem distalen Ende (15, 115) der Auslöseröhre (20, 120) zwischen dem äußersten Ende der Hohlnadel (26, 126), dem distalen Ende (15, 115) der Kanüle (14, 114) und einer zurückgezogenen Stellung mit dem distalen Ende (17, 117) der Auslöseröhre (20, 120), die zum distalen Ende (15, 115) der Hohlnadel zurückgezogen wird
aufweist.

2. Die Trokarvorrichtung (10, 110) gemäß Anspruch 1, wobei die Auslöseröhre (20, 120) so befestigt ist, dass sie mit der Schaltvorrichtung (75, 175) verbunden werden kann, wenn sie in die zurückgezogene Position bewegt wird und danach die Schaltvorrichtung (75, 175) dazu bringt, die Schließvorrichtung (66, 166) zu betätigen, wenn sie in ihre ausgeweitete Ruhestellung zurückversetzt wird.

3. Die Trokarvorrichtung (10, 110) gemäß Anspruch 2 mit einer weiteren Vorrichtung (56, 156), die die Auslöseröhre (20, 120) in ihre ausgeweitete Ruhestellung drückt.

4. Die Trokarvorrichtung (10, 110) gemäß Anspruch 1, wobei die Auslösevorrichtung (57, 55 und 71) aus der Verlängerungshohlnadel (24, 124) besteht und der Trokar zur axialen wechselseitigen Bewegung relativ zur Kanüle (24, 124) zwischen der zurückgezogenen Ruhestellung, der vollständig ausgeweiteten Projektionsstellung und einer Zwischenstellung befestigt ist, die zur vollständig ausgeweiteten Stellung zurückgezogen ist, in der der Bohrpunkt (24, 124) ebenfalls über das distale Ende (15, 115) der Kanüle (14, 114) hinaus exponiert ist; und wobei die Schaltvorrichtung (75, 175) der Trokar (24, 124) mit der Schließvorrichtung (66, 166) verbindet, wenn der Trokar (24, 124) sich von der vollständig ausgeweiteten Stellung zur Zwischenstellung bewegt und die Schließvorrichtung (66, 166) schaltet, wenn der Trokar (24, 124) in die vollständig ausgeweitete Stellung zurückkehrt, um der Trokar (24, 124) freizugeben und dabei zu ermöglichen, dass der Trokar (24, 124) mit Hilfe der Vorspannvorrichtung (56, 156) in die zurückgezogenen Ruhestellung zurückkehrt.

5. Die Trokarvorrichtung(10, 110) gemäß Anspruch 4 mit einer weiteren Vorrichtung, die den Trokar (24, 124) von der Zwischenstellung in die vollständig ausgeweitete Position drückt, wenn der Trokar (24, 124) mit der Schließvorrichtung (66, 166) verschlossen wird.

6. Die Trokarvorrichtung (10, 110) gemäß Anspruch 4, wobei der Trokar (24, 124) desweiteren ein Leerlauf-Bindeglied (98, 198) aufweist, das der Trokar (24, 124) mit der Schließvorrichtung (66, 166) verbindet und Ober- und Unterflansche beinhaltet, die am nächstgelegenen Ende der Hohlnadel (24, 124) befestigt sind, sowie eine Arretierplatte an der Hohlnadel (24, 124) für die wechselseitige Bewegung zwischen den Ober- und Unterflanschen.

7. Die Trokarvorrichtung (10, 110) gemäß Anspruch 1, wobei der Kolben eine Vorrichtung zum Greifen eines Teil des Trokar (24, 124) aufweist, der sich entfernt vom Bohrpunkt (26, 126) befindet, wenn sich der Trokar (24, 124) in der zurückgezogenen Ruhestellung befindet und zum Freigeben des entfernten Teils, wenn der Trokar (24, 124) die vollständig ausgeweitete Stellung erreicht.

8. Die Trokarvorrichtung (10, 110) gemäß Anspruch 1, wobei der Trokar (24, 124), die Vorspannvorrichtung (56, 156) und die Schließvorrichtung (75, 175) eine Trokar-Untergruppe (18, 118) bilden, die als Einheit von der Kanüle (14, 114) abgenommen werden können.

## Revendications

1. Instrument à trocart de sécurité pour percer une paroi d'une cavité anatomique possédant une partie intérieure de manière à permettre une communication avec la partie intérieure de la cavité, ledit instrument à trocart comportant :
(a) une canule tubulaire (14, 114) possédant une extrémité distale (15, 115),
(b) un trocart allongé (24, 124), ayant une pointe perçante effilée (26, 126) se terminant par une extrémité finale, protégé d'une manière réversible par un manchon (20, 120) ayant une extrémité distale (17, 117) pouvant être monté de manière à pouvoir se déplacer axialement en va-et-vient à l'intérieur de ladite canule (14, 114) entre une position de repos rétractée dans laquelle ladite pointe est reçue à l'intérieur de l'extrémité distale (15, 115) de ladite canule (14, 114) et dudit manchon (20, 120) et est protégée par ceux-ci et une position déployée dans laquelle ladite pointe (26, 126) dépasse de l'extrémité distale (15, 115) de ladite canule (14, 114) et dudit manchon (20, 120),
(c) des moyens de rappel (56, 156) pour rappeler ledit trocart (24, 124) dans sa position de repos rétractée,
(d) des moyens de verrouillage (66, 166) pour verrouiller ledit trocart (24, 124) dans la position déployée, contrairement auxdits moyens de rappel (56, 156), de manière à permettre de voir clairement, sans obstruction, la paroi de la cavité afin de pouvoir la percer avec précision,
(e) des moyens d'enclenchement (75, 175) destinés à enclencher lesdits moyens de verrouillage (66, 166) de manière à ce qu'ils déverrouillent ledit trocart (24, 124) et à permettre ainsi audit trocart (24, 124) d'être ramené dans sa position de repos rétractée par lesdits moyens de rappel (56, 156),
(f) des moyens de déclenchement mécaniques (57, 55 et 71) montés à l'intérieur dudit instrument (10, 110), comportant une partie de détection de contre-force (98, 198) qui peut être positionnée entre l'extrémité finale de ladite pointe (26, 126) du trocart et l'extrémité distale (15, 115) de ladite canule (14, 114), ladite partie de détection (98, 198) détectant une contre-force exercée par la paroi de la cavité après que celle-ci a commencé d'être percée par ladite pointe (26, 126) du trocart et détectant la diminution d'une telle contre-force lorsque ladite partie de détection (98, 198) a percé la paroi de la cavité, lesdits moyens de déclenchement (96) pouvant être accouplés mécaniquement auxdits moyens d'enclenchement (75, 175) de manière à amener lesdits moyens d'enclenchement (75, 175) à enclencher lesdits moyens de verrouillage (66, 166) en réponse à la détection de la diminution d'une telle contre-force de manière à protéger les organes internes, et
(g) des moyens formant plongeur pour déplacer manuellement ledit trocart (24, 124) à partir de la position de repos rétractée vers la position déployée,
lesdits moyens de déclenchement comportant un manchon de déclenchement tubulaire (20, 120) dans lequel est reçu d'une manière coaxiale ledit trocart (24, 124), et ladite partie de détection comportant l'extrémité distale (17, 117) dudit manchon de déclenchement (20, 120) qui peut être positionnée entre l'extrémité finale de ladite pointe (26, 126) du trocart et l'extrémité distale (15, 115) de ladite canule (14, 114), ledit manchon de déclenchement (20, 120) étant monté d'une manière coaxiale à l'intérieur de ladite canule (14, 114) de manière à pouvoir se déplacer en va-et-vient entre une position de repos déployée dans laquelle l'extrémité distale (17, 117) du manchon de déclenchement (20, 120) est positionnée entre l'extrémité finale de ladite pointe (26, 126) du trocart et l'extrémité distale (15, 115) de ladite canule (14, 114) et une position rétractée dans laquelle l'extrémité distale (17, 117) du manchon de déclenchement (20, 120) est rétractée vers l'extrémité distale (15, 115) de ladite canule (14, 114).

2. Instrument (10, 110) à trocart de sécurité selon la revendication 1, dans lequel ledit manchon de déclenchement (20, 120) est monté de manière à être accouplé auxdits moyens d'enclenchement (75, 175) lorsqu'il est déplacé vers la position rétractée et amène ensuite lesdits moyens d'enclenchement (75, 175) à enclencher lesdits moyens de verrouillage (66, 166) lorsqu'il est ramené dans sa position de repos déployée.

3. Instrument (10, 110) à trocart de sécurité selon la revendication 2, comportant en outre des moyens (56, 156) pour rappeler ledit manchon de déclenchement (20, 120) dans sa position de repos déployée.

4. Instrument (10, 110) à trocart de sécurité selon la revendication 1, dans lequel lesdits moyens de déclenchement (57, 55 et 71) sont constitués dudit trocart allongé (24, 124), ledit trocart étant monté de manière à pouvoir se déplacer en va-et-vient avec ladite canule (14, 114) entre la position de repos rétractée, la position entièrement déployée, et une position intermédiaire en retrait vers la position de repos par rapport à la position entièrement déployée dans laquelle ladite pointe (26, 126) dépasse aussi de l'extrémité distale (15, 115) de ladite canule (14, 114),
et dans lequel lesdits moyens d'enclenchement (75, 175) accouplent ledit trocart (24, 124) auxdits moyens de verrouillage (66, 166) lorsque ledit trocart (24, 124) se déplace à partir de la position entièrement déployée vers la position intermédiaire, et enclenchent lesdits moyens de verrouillage (66, 166) lorsque ledit trocart (24, 124) retourne vers ladite position entièrement déployée afin de déverrouiller ledit trocart (24, 124) et afin de permettre aussi audit trocart (24, 124) d'être déplacé dans la position de repos rétractée par lesdits moyens de rappel (56, 156).

5. Instrument (10, 110) à trocart de sécurité selon la revendication 4, comportant en outre des moyens pour rappeler ledit trocart (24, 124) à partir de la position intermédiaire vers la position entièrement déployée lorsque ledit trocart (24, 124) est verrouillé sur lesdits moyens de verrouillage (66, 166).

6. Instrument (10, 110) à trocart de sécurité selon la revendication 4, dans lequel ledit trocart (24, 124) comporte en outre des moyens d'accouplement à course perdue (98, 198) pour accoupler ledit trocart (24, 124) auxdits moyens de verrouillage (66, 166) et incluant des rebords supérieur et inférieur montés dans la région de l'extrémité proximale dudit trocart (24, 124) et une plaque d'arrêt montée sur ledit trocart (24, 124) pour un déplacement en va-et-vient entre lesdits rebords supérieur et inférieur.

7. Instrument (10, 110) à trocart de sécurité selon la revendication 1, dans lequel lesdits moyens formant plongeur comportent des moyens pour saisir une partie dudit trocart (24, 124) qui est éloignée de ladite pointe perçante (26, 126) lorsque ledit trocart (24, 124) se situe dans la position de repos rétractée, et pour libérer ladite partie éloignée lorsque ledit trocart (24, 124) atteint la position entièrement déployée.

8. Instrument (10, 110) à trocart de sécurité selon la revendication 1, dans lequel ledit trocart (24, 124), lesdits moyens de rappel (56, 156), et lesdits moyens d'enclenchement (75, 175) constituent un sous-ensemble à trocart (18, 118) qui peut être enlevé de ladite canule (14, 114) en un seul bloc.
